# FASCICULE DE BREVET EUROPEEN

(11) **EP 0 725 601 B1**
(45) Date de publication et mention de la délivrance du brevet: **31.07.2002**
(21) Numéro de dépôt: 95928540.4
(22) Date de dépôt: 24.08.1995
(51) Int. Cl.: A61B 17/84, A61B 17/70

(54) **BOUCHON FILETE DE SERRAGE D'UNE INSTRUMENTATION D'OSTEOSYNTHESE**
SCHRAUBKLAPPE ZUR BEFESTIGUNG EINER OSTEOSYNTHETISCHEN EINRICHTUNG
OSTEOSYNTHESIS INSTRUMENT

(30) Priorité: 29.08.1994 FR 9410377
(43) Date de publication de la demande: 14.08.1996
(73) Titulaire: SOCIETE DE FABRICATION DE MATERIEL ORTHOPEDIQUE SOFAMOR, 93290 Tremblay-en-France (FR)
(72) Inventeur: GOURNAY, José, F-62600 Berk Plage (FR); PETIT, Dominique, F-62600 Berk-sur-Mer (FR); SAURAT, Jean, F-62630 Etaples (FR)
(74) Mandataire: Neyret, Daniel
(86) Numéro de dépôt international: FR9501115
(87) Numéro de publication internationale: WO9606576

(56) Documents cités:
- EP-A- 0 172 130
- EP-A- 0 195 455
- EP-A- 0 276 153
- EP-A- 0 348 272
- EP-A- 0 465 158
- WO-A-92/03100
- WO-A-94/10927
- WO-A-94/10944
- DE-A- 3 630 863
- US-A- 4 790 297

## Description

La présente invention a pour objet un bouchon fileté de serrage adapté pour assurer une liaison entre deux implants d'une instrumentation d'ostéosynthèse. rachidienne ou autres, du type visé au préambule de la revendication 1.

Dans les instrumentations d'ostéosynthèse rachidienne utilisées jusqu'à présent, notamment celles du type "COTREL-DUBOUSSET" (brevets américains 4641636 et 4815453), les éléments constitutifs sont mis en place et assemblés au moyen de petites vis. En fin de vissage, le chirurgien provoque leur rupture afin d'enlever leur tête, ce qui exige de couper les tiges pour procéder, si nécessaire, à une ablation ultérieure de l'instrumentation, ainsi rendue relativement malaisée.

De plus, la mesure des couples de serrage des vis nécessite des instruments supplémentaires dont la précision n'est pas toujours satisfaisante.

Le document D1 (WO-A94/10927) décrit une instrumentation d'ostéosynthèse rachidienne comprenant des vis de liaison entre deux implants, formées de deux parties coaxiales séparées par une ligne d'amorce de rupture. La seconde partie de chaque vis est vissée à l'aide d'une empreinte de la première partie.

Toutefois avec ces vis la rupture entre les deux parties n'intervient pas à un couple bien déterminé et de plus la seconde partie ne peut plus être desserrée après la rupture.

Le document D2 (EP-A-0 654 248) divulgue une vis à os présentant une ligne d'amorce de rupture et une empreinte de la tige filetée, accessible en cas de rupture accidentelle de la tête de la vis. Il s'agit donc ici de réaliser une sécurité permettant le dévissage de la vis uniquement en cas de rupture lors du vissage, ce qui ne survient que de manière accidentelle.

L'invention a pour buts, d'une part de réaliser un élément d'assemblage permettant de démonter ultérieurement de manière plus aisée l'instrumentation en cas de besoin grâce à un dévissage de la seconde partie filetée du bouchon, et d'autre part, de réaliser un système intégré, au bouchon, assurant un couple de serrage prédéterminé précis.

Conformément à l'invention, le bouchon fileté est réalisé selon la partie caractérisante de la revendication 1.

La partie, filetée peut être vissée dans un trou taraudé ménagé dans l'implant correspondant, ou sur le filetage du corps d'un organe d'ancrage vertébral (vis ou crochet) pour assurer la liaison de cet organe avec une tige, ou sur une tige filetée d'un dispositif de liaison transversale entre deux tiges longitudinales d'ostéosynthèse.

Bien entendu, ces trois exemples de mise en oeuvre du bouchon selon l'invention ne sont pas limitatifs, l'invention ayant une portée très générale dans le cadre d'instrumentations d'ostéosynthèse.

Ainsi, après cassure, lorsque le couple prévu est dépassé, et séparation des deux parties, la seconde partie peut, si nécessaire, être desserrée, grâce à son empreinte, adaptée à un outil approprié, ce qui permet le démontage du dispositif.

La valeur du couple de rupture est fonction de la profondeur de la ligne d'amorce de rupture, et peut être aisément ajustée à la grandeur souhaitée. Il est donc possible d'obtenir pour le couple de serrage une précision accrue par rapport aux couples de serrage mis en oeuvre jusqu'à présent avec les vis habituelles, car il est possible de déterminer le couple de rupture de manière beaucoup plus précise qu'avec les moyens habituels qui impliquent l'utilisation d'un couplemètre relativement peu précis.

D'autres particularités et avantages de l'invention apparaîtront au cours de la description qui va suivre, faite en référence aux dessins annexés qui en illustrent plusieurs formes de réalisation à titre d'exemples non limitatifs.

La figure 1 est une vue en élévation à échelle agrandie d'une première forme de réalisation du bouchon fileté selon l'invention.

La figure 2 est une vue de dessus du bouchon de la figure 1.

La figure 3 est une vue en élévation partielle à échelle agrandie du détail A de la figure 1.

La figure 4 est une vue en élévation à échelle agrandie d'une seconde forme de réalisation du bouchon conforme à l'invention.

La figure 5 est une vue de dessus du bouchon de la figure 4.

La figure 6 est une vue de dessous du bouchon de la figure 4.

La figure 7 est une vue en élévation à échelle agrandie d'une troisième forme de réalisation du bouchon fileté selon l'invention.

La figure 8 est une vue de dessus du bouchon de la figure 7.

La figure 9 est une vue en élévation à échelle agrandie d'une quatrième forme de réalisation du bouchon selon l'invention.

La figure 10 est une vue de dessus du bouchon de la figure 9.

La figure 11 est une vue en perspective partielle d'une instrumentation illustrant un exemple de mise en oeuvre du bouchon fileté selon l'invention.

La figure 12 est une vue en élévation et coupe partielle, à échelle agrandie, d'une cinquième forme de réalisation du bouchon selon l'invention et d'une instrumentation correspondante de mise en oeuvre.

La figure 13 est une vue en élévation et coupe partielle, à échelle agrandie, d'un troisième exemple de mise en oeuvre du bouchon selon l'invention.

La figure 14 est une variante d'exécution du bouchon de la figure 13.

La figure 15 illustre une variante d'exécution de la réalisation de la figure 12.

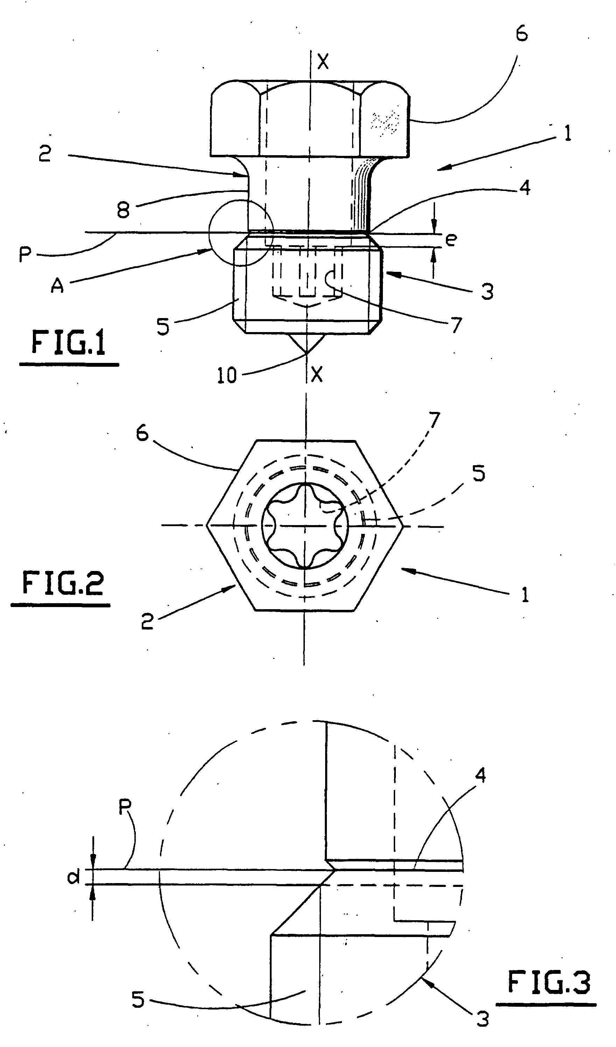

En se reportant aux figures 1 à 3, on voit un bouchon 1 de serrage destiné à assurer une liaison entre deux implants constitutifs d'une instrumentation d'ostéosynthèse rachidiennne non représentée, par exemple du type de celle illustrée dans les brevets précités.

Le bouchon 1 est constitué de deux parties coaxiales 2,3 séparées par une ligne 4 d'amorce de rupture. La partie 1, non filetée, constitue la tête de serrage du bouchon, tandis que la partie 3 est pourvue d'un filetage extérieur 5 et destinée à venir se noyer dans un élément à assembler à un autre sur lequel la partie filetée 3 doit être serrée.

La première partie 1 est pourvue d'une empreinte extérieure hexagonale 6 d'axe (XX), lequel est l'axe général du bouchon 1. Une seconde empreinte 7, différente de l'empreinte 6, hexalobée dans l'exemple représenté et coaxiale à l'empreinte hexagonale 6, est formée dans la seconde partie 3, au-delà de la ligne de rupture 4.

L'empreinte 6 est raccordée à une partie cylindrique lisse 8 de liaison avec la seconde partie 3, la ligne 4 d'amorce de rupture étant usinée à l'extrémité de la partie 8. Un écart prédéterminé e est réservé entre le plan P de la ligne de rupture 4 et le début de l'empreinte 7 de la seconde partie 3, laquelle est dimensionnée pour pouvoir être noyée dans l'implant en fin de vissage. Par ailleurs, le filetage 5 de la seconde partie 3 s'arrête juste avant la ligne 4 d'amorce de rupture, dont il est séparé par une distance d (fig. 3). La ligne 4 d'amorce de rupture est constituée par une saignée de profondeur correspondant à la valeur prédéterminée du couple de rupture. Ce dernier peut avantageusement être prévu, ainsi que l'empreinte 7 de desserrage de la seconde partie 3, pour que cette empreinte 7 ne puisse résister à un couple de serrage supérieur au couple de rupture, et ce, afin d'empêcher toute tentative de poursuite du serrage de la partie 3 après rupture du bouchon.

La mise en oeuvre du bouchon 1 qui vient d'être décrit résulte directement de la description ci-dessus. Le vissage de la partie 3 dans l'implant est effectué au moyen d'un outil non représenté coopérant avec l'empreinte hexagonale 6, la rupture entre les deux parties 1 et 3 ayant lieu au niveau de la ligne d'amorce 4 dès que le couple de serrage maximum prévu est atteint.

Le décalage e entre le plan P de la ligne de rupture 4 et le début de l'empreinte 7 de la partie 3 de serrage présente l'avantage d'empêcher l'empreinte 7 d'être endommagée à la rupture, comme cela serait le cas si aucun intervalle entre le plan P et le début de l'empreinte 7 n'était prévu. Ainsi peut être obtenue une rupture franche de la tête du bouchon, laissant apparaître une surface lisse à couple constant et non agressive pour les intervenants.

Par ailleurs, le fait que le filet 5 de la vis de desserrage s'arrête juste avant la ligne 4 d'amorce de rupture, évite la formation, à la rupture, d'une bavure sur le début du filet 5. Il importe en effet d'éviter une telle bavure, douloureuse pour le patient, qui pourrait endommager les gants du chirurgien, et d'obtenir au contraire une rupture lisse et propre.

Le contour hexalobé de l'empreinte intérieure 7 est particulièrement avantageux par rapport à d'autres types d'empreinte, en raison de sa résistance élevée qui évite sa déformation. D'une manière générale, des empreintes de révolution telles que 6 et 7 sont préférables à de simples fentes de tournevis, en raison de leur résistance plus élevée à l'effort exercé par l'outil.

La précision sur le couple de rupture peut être d'environ ± 3% en fonction des tolérances d'usinage. La valeur de ce couple et la précision sur celui-ci sont obtenues en approfondissant plus ou moins, la saignée d'amorce de rupture.

Cette précision est largement supérieure à celle obtenue jusqu'à présent avec les vis habituelles, en utilisant des couples mètres.

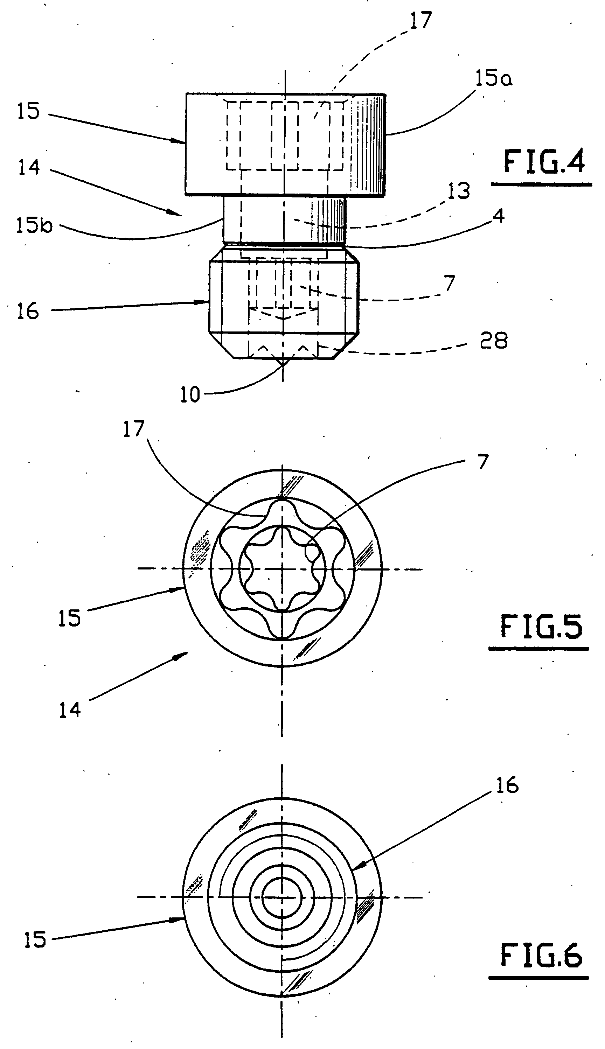

Dans le second mode de réalisation, illustré aux figures 4 à 6, le bouchon 14 est constitué comme le précédent en deux parties 15 et 16. Il en diffère par le fait que l'empreinte 17 de la partie 15 de serrage est intérieure et hexalobée, comme l'empreinte 7 de la partie de desserrage filetée 16, la surface extérieure 15a de la tète 15 étant lisse. Cette tête 15 est reliée par une partie cylindrique lisse 15b à la partie filetée 16.

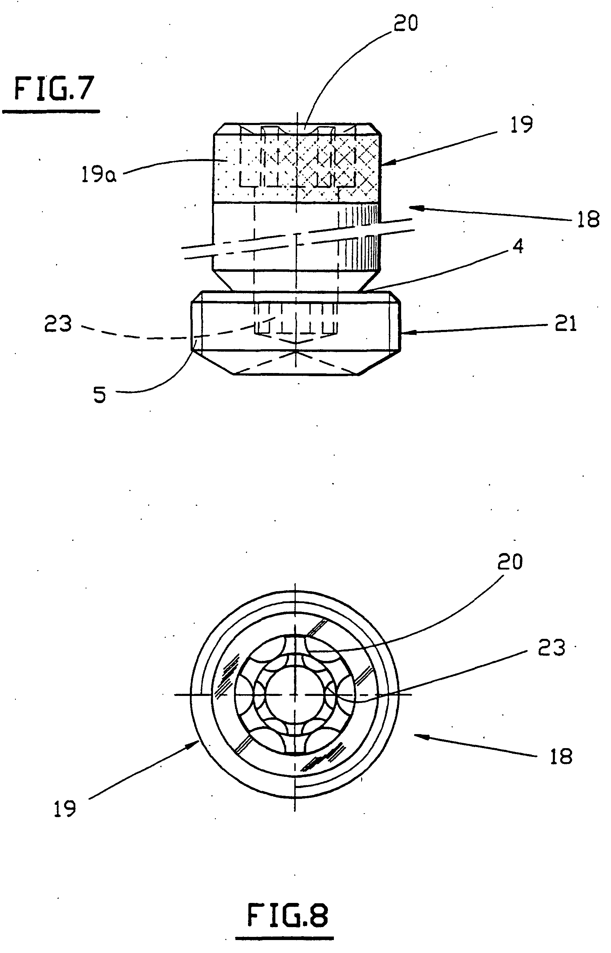

Dans la troisième forme d'exécution, illustrée aux figures 7 et 8 , le bouchon 18, formé de deux parties 19, 21 séparées par la ligne d'amorce 4 de rupture, comporte deux empreintes intérieures coaxiales 20 et 23, respectivement dans la partie de serrage 19 et la partie de desserrage 21. La partie 19 peut porter un moletage 19a permettant la mise en place et un serrage manuels du bouchon 18. En fonction de la valeur du couple de rupture prévu, la mise en place et le serrage du bouchon 18 peuvent être, soit exclusivement manuels au moyen de la partie moletée 19a (ou autre), soit complétée par un serrage mécanique au moyen de l'empreinte 20.

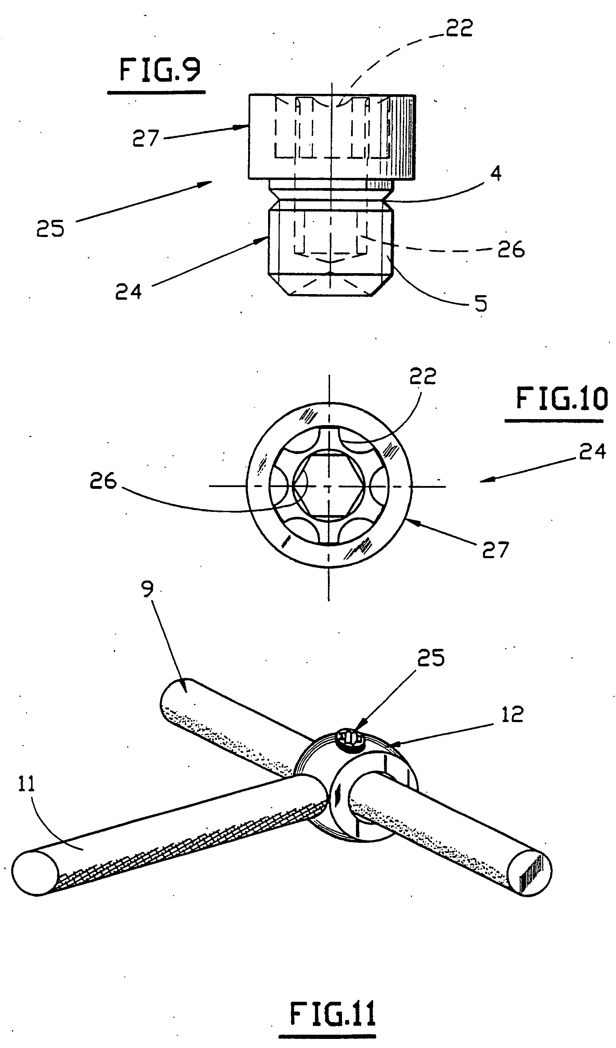

La quatrième forme d'exécution illustrée aux figures 9 et 10, diffère de celle des figures 7 et 8 essentiellement en ce que dans la partie de desserrage 24 du bouchon 25, est ménagée une empreinte hexagonale 26, coaxiale à l'empreinte hexalobée 22 de la partie de serrage 27.

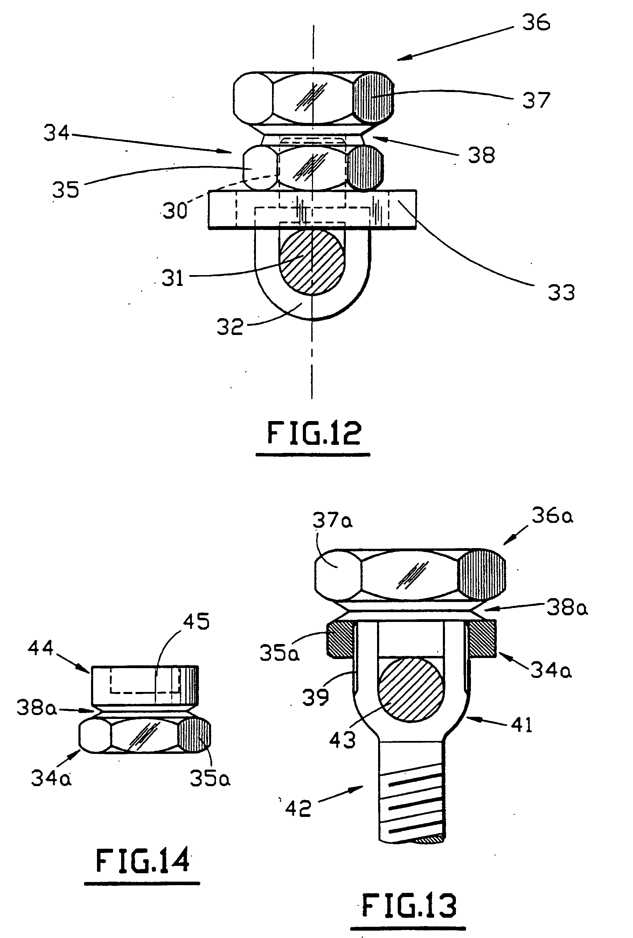

La figure 11 illustre un exemple de mise en oeuvre du bouchon fileté 1 selon l'invention. On voit sur cette figure 11 une tige 9 d'ostéosynthèse, associée à un bras latéral 11 à l'extrémité duquel est fixée une bague 12 traversée par la tige 1. La tige 9 peut avoir une surface quelconque : lisse, microbillée, à aspérités, filetée... Dans la bague 12, est agencé un trou taraudé permettant de recevoir un bouchon fileté, tel que 25, pour l'assemblage de la tige 9 et du bras 11. Ce bouchon 25 (ou l'un des autres décrits), remplace avantageusement les vis classiques utilisées comme décrit par le brevet français précité 92 13476. Après séparation de sa partie 27, sa partie restante 24 ne dépasse pas de l'implant. Ainsi, le bouchon selon l'invention garantit avantageusement un faible encombrement de l'instrumentation, car il n'est jamais en relief par rapport à la pièce dans laquelle il est vissé.

La figure 12 montre un autre exemple de mise en oeuvre de l'invention dans lequel l'implant sur lequel est vissée ladite seconde partie est une tige filetée 30 d'un dispositif de liaison transversale entre deux tiges longitudinales d'ostéosynthèse, dont l'une seulement 31 est représentée.

Ce dispositif comporte un connecteur 32 traversé par la tige associée 31 et solidaire de la tige filetée 30 et une plaque 33 de liaison transversale entre les tiges 31, également traversée par la tige filetée 30. Sur cette dernière peut être vissé un écrou mâle 34 formant ladite seconde partie de desserrage et ayant une empreinte mâle 35 quelconque (hexagonale, carrée, triangulaire, encoches...). L'écrou 34 est solidaire d'un écrou de serrage 36 ayant une empreinte d'entraînement quelconque, mâle ou femelle : dans l'exemple représenté, cet entraînement est mâle grâce à une empreinte extérieure hexagonale 37. Les deux écrous 34, 36 sont reliés par une zone 38 d'amorce de rupture similaire aux précédentes. Ce dispositif de liaison transversale (32, 33, 34) est illustré à titre d'exemple non limitatif.

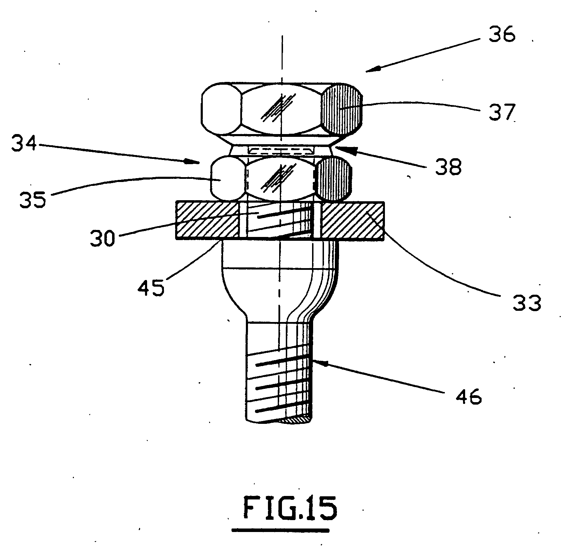

A titre de variante possible (Fig. 15), la plaque 33 peut s'appuyer sur un épaulement 45 d'une vis à os 46, le bouchon 36, 34 permettant alors de solidariser ces deux pièces.

La figure 13 montre un autre exemple de mise en oeuvre de l'invention, dans lequel le bouchon (36a, 34a), similaire au bouchon (34, 36) de la figure 12 (références 37a, 34a, 38a, 35a), a son écrou cassant 34a vissé sur le filetage 39 du corps 41 en U d'une vis à os 42 (ou d'un crochet). Après serrage et rupture de la zone 38a, l'écrou 34a assure alors la solidarisation de la vis 42 (ou crochet) avec une tige 43 reçue dans le corps 41.

Dans la variante de la figure 14, l'écrou mâle 36a est remplacé par un écrou 44 à empreinte 45 de profil quelconque.

Bien entendu l'empreinte de desserrage 35a peut être de forme quelconque.

Dans les différents modes de réalisation de l'invention, le couple de serrage obtenu est conforme aux souhaits du fabricant, et permet d'assurer la répétitivité de ce couple, que n'offrent pas les moyens de serrage dynamomètriques utilisés couramment, car la dispersion s'accroît au fur et à mesure des stérilisations de ces instruments.

D'une manière générale, tout type d'empreinte peut être utilisé dans les deux parties constitutives du bouchon, avec cependant une préférence pour les empreintes hexalobées. Il est ainsi possible de réaliser des empreintes carrées, triangulaires, cruciformes etc..., aussi bien de serrage que de démontage, intérieures et extérieures, ainsi que tout type de profil d'amorce de rupture.

L'empreinte de démontage (7, 23...) peut être borgne comme représenté, ou débouchante sur la face de ladite seconde partie tournée vers la tige de l'instrumentation. Une telle empreinte est représentée à la figure 4 qui montre une empreinte débouchante 28, le pointeau 10 étant alors supprimé. Elle permet ainsi de réaliser tout type d'interface bouchon-tige (pointeau tel que 10 sur la Fig. 1), cuvette etc...

Le bouchon peut avoir un diamètre quelconque et casser pour tout couple prédéterminé.

Avantageusement, on peut utiliser une partie standard de serrage identique pour différents types de partie filetée de desserrage, ce qui simplifie la fabrication du bouchon et en diminue le prix de revient. On peut aussi superposer deux empreintes identiques séparées par une ligne d'amorce de rupture. La première partie (2...) peut avoir une forme quelconque, en creux ou en relief, être par exemple filetée.

Il est possible de prévoir avantageusement l'utilisation d'un instrument permettant de retenir la tête (première partie) du bouchon une fois cassée, ou encore un système de maintien de la tête sur le tournevis empêchant la tête de tomber dans le champ opératoire.

La tête du bouchon peut avoir une géométrie particulière permettant d'assurer un maintien au moyen de serrage, un maintien de la tête cassée, et une mise en place manuelle (Fig. 7). On peut utiliser la première partie du bouchon comme point d'ancrage d'une instrumentation permettant une liaison entre implants à des fins de correction, ou de distraction, ou de. compression. Enfin, contrairement à ce qui est illustré aux dessins, notamment à la figure 4, où les deux empreintes 17 et 7 débouchent dans une chambre intermédiaire 13, il est possible d'obturer cette chambre. Dans ce cas, l'empreinte 7 de la partie de serrage 16 est alors remplacée par la partie débouchante 28.

## Revendications

1. Bouchon fileté (1 ; 14 ; 18 ; 25) de serrage destiné à assurer une liaison entre deux implants d'une instrumentation d'ostéosynthèse, ledit bouchon étant constitué de deux parties coaxiales (2, 3 ;15, 16 ; 19, 21 ; 24, 27) séparées par une ligne (4) d'amorce de rupture, la première partie (2 ; 15 ;19 ; 27) étant pourvue d'une empreinte (6 ; 17 ;20 ;22 ;37) destinée à permettre le vissage de la seconde partie filetée (3 ; 16 ; 21 ;24) sur un implant jusqu'à un couple de rupture prédéterminé entre les deux parties (2, 3 ;15, 16 ; 19, 21 ; 24, 27), **caractérisé en ce que** la seconde partie filetée (3 ; 16 ; 21 ;24) est pourvue d'une empreinte (7; 23 ;26 ;35) coaxiale à l'empreinte (6 ; 17 ;20 ;22 ;37) de la première partie (2 ; 15 ; 19 ; 27) permettant un desserrage éventuel ultérieur de cette seconde partie (3 ; 16 ; 21 ; 24), et **en ce qu'**un écart déterminé (e) est réservé entre le plan (P) de la ligne (4) d'amorce de rupture et le début de l'empreinte (7; 23 ;26 ;35) de la seconde partie (3 ; 16 ; 21 ;24).

2. Bouchon selon la revendication 1, **caractérisé en ce que** ladite seconde partie filetée (3 ; 16...) peut être vissée dans un trou taraudé ménagé dans l'un (12) des implants.

3. Bouchon selon la revendication 1, **caractérisé en ce que** le filetage (5) de la seconde partie (3 ; 16 ; 21 ; 24) s'arrête juste avant la ligne (4) d'amorce de rupture.

4. Bouchon selon la revendication 1, **caractérisé en ce que** ladite seconde partie filetée (3 ; 16 ; 21 ; 24) est dimensionnée pour être noyée dans l'implant en fin de vissage.

5. Bouchon selon la revendication 2, **caractérisé en ce que** la ligne d'amorce de rupture (4) est constituée par une saignée de profondeur correspondant à la valeur prédéterminée du couple de rupture lequel est prévu, ainsi que l'empreinte de desserrage (7 ; 23) de la seconde partie (3 ; 16...) pour que ladite empreinte ne puisse résister à un couple de serrage supérieur au couple de rupture.

6. Bouchon selon la revendication 2, **caractérisé en ce que** l'empreinte (7 ; 20 ; 22) de la première partie (15 ; 19 ; 27) est intérieure.

7. Bouchon selon la revendication 6, **caractérisé en ce que** ladite empreinte (7 ; 20 ; 22) est hexalobée.

8. Bouchon selon la revendication 2, **caractérisé en ce que** l'empreinte de la première partie (2) est extérieure, par exemple polygonale (6).

9. Bouchon selon la revendication 2, **caractérisé en ce que** l'empreinte de la seconde partie (3 ; 16 ; 21) est intérieure et hexalobée (7).

10. Bouchon (25) selon la revendication 9, **caractérisé en ce que** l'empreinte de la seconde partie (26) est hexagonale.

11. instrumentation d'ostéosynthèse comprenant au moins une tige (9) adaptée à coopérer avec un bouchon fileté de serrage (1 ; 14 ; 18 ; 25), **caractérisée en ce que** ledit bouchon (1 ; 14 ; 18 ; 25) est du type selon la revendication 1, et **en ce que** l'empreinte (7 ; 23 ; 26) de ladite seconde partie est borgne ou débouchante sur la face de ladite seconde partie filetée (3 ; 16 ; 21 ; 24) tournée vers la tige (9) de l'instrumentation.

12. Instrumentation d'ostéosynthèse comprenant deux tiges longitudinales (31) et une tige filetée (30) d'un dispositif de liaison transversale entre les deux tiges longitudinales, ladite instrumentation comprenant également un bouchon fileté (1, 14, 18, 25) de serrage destiné à être vissé sur ladite tige filetée (30), **caractérisée en ce que** ledit bouchon (1 ; 14 ;18 ;25) est du type selon la revendication 1, et **en ce que** ladite tige filetée (30) constitue un implant sur lequel est vissée ladite seconde partie, laquelle est un écrou mâle (34) de serrage pourvu d'une empreinte (35) de forme quelconque.

## Patentansprüche

1. Schraubverbindung (1 ; 14; 18 ; 25), zur Herstellung einer Verbindung zwischen zwei Implantaten einer Osteosynthesevorrichtung, wobei die Schraubverbindung aus zwei koaxialen Teilen (2, 3 ; 15, 16 ; 19, 21; 24, 27) besteht, die durch eine Anbruchlinie (4) getrennt sind, und wobei der erste Teil (2 ; 15 ; 19 ; 27) mit einer Prägung (6 ; 17; 20 ; 22 ; 37) zum Schrauben des zweiten Gewindeteils (3 ; 16; 21 ; 24) auf ein Implantat bis zu einem vorbestimmten Bruchmoment zwischen den beiden Teilen (2, 3 ; 15, 16 ; 19, 21 ; 24, 27) versehen ist, **dadurch gekennzeichnet, daß** der zweite Gewindeteil (3 ; 16 ; 21 ; 24) mit einer zur Prägung (6 ; 17 ; 20; 22 ; 37) des ersten Teils (2 ; 15 ; 19 ; 27) koaxialen Prägung (7 ; 23; 26 ; 35) für ein eventuelles Lösen des zweiten Teils (3 ; 16 ; 21 ; 24) versehen ist, und daß ein bestimmter Abstand (e) zwischen der Ebene (P) der Anbruchlinie (4) und dem Anfang der Prägung (7 ; 23 ; 26 ; 35) des zweiten Teils (3 ; 16 ; 21; 24) gelassen ist.

2. Schraubverbindung nach Anspruch 1, **dadurch gekennzeichnet, daß** der besagte zweite Gewindeteil (3 ; 16; ...) in ein in eines (12) der Implantate eingearbeitetes Gewindeloch einschraubbar ist.

3. Schraubverbindung nach Anspruch 1, **dadurch gekennzeichnet, daß** das Gewinde (5) des zweiten Teils (3 ; 16 ; 21; 24) gerade vor der Anbruchlinie (4) aufhört.

4. Schraubverbindung nach Anspruch 1, **dadurch gekennzeichnet, daß** der besagte zweite Gewindeteil (3 ; 16; 21 ; 24) so bemessen ist, daß er, wenn das Schrauben beendet ist, im Implantat versenkt ist .

5. Schraubverbindung nach Anspruch 2, **dadurch gekennzeichnet, daß** die Anbruchlinie (4) aus einem Schnitt besteht, dessen Tiefe dem vorbestimmten Wert des Bruchmoments entspricht und der ebenso wie die Prägung (7; 23) des zweiten Teils (3 ; 16 ; ...) so konzipiert ist, daß die besagte Prägung einem Anzugsmoment, das größer als das Bruchmoment ist, nicht widersteht.

6. Schraubverbindung nach Anspruch 2, **dadurch gekennzeichnet, daß** die Prägung (7 ; 20 ; 22) des ersten Teils (15 ; 19 ; 27) sich innen befindet.

7. Schraubverbindung nach Anspruch 6, **dadurch gekennzeichnet, daß** die besagte Prägung (7 ; 20; 22) sechslappig ist.

8. Schraubverbindung nach Anspruch 2, **dadurch gekennzeichnet**« daß die Prägung des ersten Teils (2) sich außen befindet und z.B. polygonal (6) ist.

9. Schraubverbindung nach Anspruch 2, **dadurch gekennzeichnet, daß** die Prägung des zweiten Teils (3 ; 16 ; 21) sich innen befindet und sechslappig (7) ist.

10. Schraubverbindung (25) nach Anspruch 9, **dadurch gekennzeichnet, daß** die Prägung des zweiten Teils (26) sechseckig ist.

11. Osteosynthesevorrichtung mit mindestens einem Stift (9) zum Zusammenwirken mit einer Schraubverbindung (1 ; 14 ; 18; 25), **dadurch gekennzeichnet, daß** die Verbindung (1 ; 14 ; 18 ; 25) vom Typ des Anspruchs 1 ist, und daß die Prägung (7 ; 23 ; 26) des zweiten Teils blind ist oder auf der Seite des zweiten Gewindeteils (3 ; 16 ; 21; 24) mündet, die zum Stift (9) der Vorrichtung gewendet ist.

12. Osteosynthesevorrichtung mit zwei Längsstiften (31) und einem Gewindestift (30) einer transversalen Verbindungsvorrichtung zwischen den beiden Längsstiften, wobei die Osteosynthesevorrichtung ebenfalls eine Schraubverbindung (1 ; 14 ; 18 ; 25) zum Schrauben auf den Gewindestift (30) aufweist, **dadurch gekennzeichnet, daß** die Schraubverbindung (1 ; 14; 18 ; 25) vom Typ des Anspruchs 1 ist, und daß der Gewindestift (30) ein Implantat darstellt, auf das der zweite Teil schraubbar ist, der eine Steckmutter (34) ist, die mit einer beliebig geformten Prägung (35) versehen ist.

## Claims

1. A screwthreaded locking plug (1; 14; 18; 25) intended to provide a connection between two implants of an osteosynthesis instrument, said plug being formed by two coaxial parts (2, 3; 15, 16; 19, 21; 24, 27) separated by a rupture start line (4), the first part (2; 15; 19; 27) being provided with an engagement means (6; 17, 20; 22; 37) intended to permit screwing of the second screwthreaded part (3; 16; 21; 24) on to an implant up to a predetermined rupture torque between the two parts (2, 3; 15, 16; 19, 21; 24, 27), **characterised in that** the second screwthreaded part (3; 16; 21; 24) is provided with an engagement means (7; 23; 26; 35) coaxial with the engagement means (6; 17; 20; 22; 37) of the first part (2; 15; 19; 27) permitting subsequent release if required of said second part (3; 16; 21; 24), and that a given spacing (e) is reserved between the plane (P) of the rupture start line (4) and the beginning of the engagement means (7; 23; 26; 35) in the second part (3; 16; 21; 24).

2. A plug according to claim 1 **characterised in that** said second screwthreaded part (3; 16...) can be screwed into a threaded hole provided in one (12) of the implants.

3. A plug according to claim 1 **characterised in that** the screwthread (5) of the second part (3; 16; 21; 24) stops just before the rupture start line (4).

4. A plug according to claim 1 **characterised in that** said second screwthreaded part (3; 16; 21; 24) is dimensioned to be embedded in the implant at the end of screwing.

5. A plug according to claim 2 **characterised in that** the rupture start line (4) is formed by a groove of a depth corresponding to the predetermined value of the rupture torque which is provided, and the release engagement means (7; 23) of the second part (3; 16...) so that said engagement means cannot withstand a locking torque higher than the rupture torque.

6. A plug according to claim 2 **characterised in that** the engagement means (7; 20; 22) of the first part (15; 19; 27) is internal.

7. A plug according to claim 6 **characterised in that** said engagement means (7; 20; 22) has six lobes.

8. A plug according to claim 2 **characterised in that** the engagement means of the first part (2) is external, for example polygonal (6).

9. A plug according to claim 2 **characterised in that** the engagement means of the second part (3; 16; 21) is internal and has six lobes (7).

10. A plug (25) according to claim 9 **characterised in that** the engagement means of the second part (26) is hexagonal.

11. An osteosynthesis instrument comprising at least one rod (9) adapted to co-operate with a screwthreaded locking plug (1; 14; 18; 25) **characterised in that** said plug (1; 14; 18; 25) is of the type according to claim 1 and that the engagement means (7; 23; 26) of said second part is blind or opens on the face of said second screwthreaded part (3; 16; 21; 24) which is turned towards the rod (9) of the instrument.

12. An osteosynthesis instrument comprising two longitudinal rods (31) and a screwthreaded rod (30) of a transverse connecting arrangement between the two longitudinal rods, said instrument also comprising a screwthreaded locking plug (1, 14, 18, 25) intended to be screwed on to said screwthreaded rod (30), **characterised in that** said plug (1; 14; 18; 25) is of the type according to claim 1 and that said screwthreaded rod (30) constitutes an implant on to which is screwed said second part which is a male locking nut (34) provided with an engagement means (35) of any form.
